# EUROPEAN PATENT SPECIFICATION

(11) **EP 2 356 218 B1**
(45) Date of publication and mention of the grant of the patent: **03.05.2017**
(21) Application number: 09764840.6
(22) Date of filing: 07.12.2009
(51) Int. Cl.: C12N 5/07, C12N 5/0797, A61K 35/30, C12N 5/0793, G01N 33/50

(54) **METHOD AND MEDIUM FOR NEURAL DIFFERENTIATION OF PLURIPOTENT CELLS**
VERFAHREN UND MEDIUM ZUR NEURALEN DIFFERENZIERUNG VON PLURIPOTENTEN ZELLEN
PROCÉDÉ ET MILIEU POUR LA DIFFÉRENCIATION NEURALE DE CELLULES PLURIPOTENTES

(30) Priority: 05.12.2008 EP 08305887
(43) Date of publication of application: 17.08.2011
(73) Proprietor: INSERM (Institut National de la Santé et de la Recherche Médicale), 75013 Paris (FR); Association Francaise Contre Les Myopathies, 75013 Paris (FR)
(72) Inventor: BENCHOUA, Alexandra, 91030 Evry (FR); PERRIER, Anselme, 91030 Evry (FR); AUBRY, Laetitia, 91030 Evry (FR)
(74) Representative: Cabinet Plasseraud
(86) International application number: PCT/EP2009/066504
(87) International publication number: WO 2010/063848

(56) References cited:
- WO-A-2008/056166
- NISHIKAWA SHIN-ICHI ET AL: "The promise of human induced pluripotent stem cells for research and therapy" NATURE REVIEWS MOLECULAR CELL BIOLOGY, vol. 9, no. 9, September 2008 (2008-09), pages 725-729, XP002520703 ISSN: 1471-0072
- SMITH ET AL: "Inhibition of Activin/Nodal signaling promotes specification of human embryonic stem cells into neuroectoderm" DEVELOPMENTAL BIOLOGY, ACADEMIC PRESS, NEW YORK, NY, US, vol. 313, no. 1, 11 October 2007 (2007-10-11), pages 107-117, XP022400293 ISSN: 0012-1606
- STERN CLAUDIO D: "Neural induction: old problem, new findings, yet more questions." DEVELOPMENT (CAMBRIDGE, ENGLAND) MAY 2005, vol. 132, no. 9, May 2005 (2005-05), pages 2007-2021, XP002520704 ISSN: 0950-1991
- GAMER ET AL: "BMP-3 is a novel inhibitor of both activin and BMP-4 signaling in Xenopus embryos" DEVELOPMENTAL BIOLOGY, ACADEMIC PRESS, NEW YORK, NY, US, vol. 285, no. 1, 1 September 2005 (2005-09-01), pages 156-168, XP005015206 ISSN: 0012-1606

### SEQUENCE LISTING

<110> INSERM
<120> Method and medium for neural differentiation of pluripotent cells
<130> BIO08655 BENCHOUA
<160> 8
<170> PatentIn version 3.3
<210> 1
   <211> 20
   <212> DNA
   <213> artificial
<220>
   <223> primer
<400> 1
   ctccatgaac atgcaacctg 20
<210> 2
   <211> 20
   <212> DNA
   <213> artificial
<220>
   <223> primer
<400> 2
   ctcgctgatt aggctccaac 20
<210> 3
   <211> 25
   <212> DNA
   <213> artificial
<220>
   <223> primer
<400> 3
   cttgctgcag aagtgggtgg aggaa 25
<210> 4
   <211> 21
   <212> DNA
   <213> artificial
<220>
   <223> primer
<400> 4
   ctgcagtgtg ggtttcgggc a 21
<210> 5
   <211> 20
   <212> DNA
   <213> artificial
<220>
   <223> primer
<400> 5
   gatgcacaac tcggagatca 20
<210> 6
   <211> 20
   <212> DNA
   <213> artificial
<220>
   <223> primer
<400> 6
   gtccttcttg agcagcgtct 20
<210> 7
   <211> 20
   <212> DNA
   <213> artificial
<220>
   <223> primer
<400> 7
   gccagcaaca cacctagtca 20
<210> 8
   <211> 20
   <212> DNA
   <213> artificial
<220>
   <223> primer
<400> 8
   tgtgagggct gtgtctgttc 20

## Description

### FIELD OF THE INVENTION

The invention relates to a method and a medium for the synchronous neural differentiation of pluripotent cells, in particular human pluripotent cells.

### BACKGROUND OF THE INVENTION

Stem cells, in particular human embryonic stems cells (hES cells), present two advantages due to their intrinsic properties: firstly they can provide a quasi-unlimited pool of cells due to their self-renewal capacity, and secondly, they are capable of differentiating *in vitro* into any cell lineage, including all the neural lineage cells types.

Production of neuronal and glial cells from hES cells promises to be an invaluable tool for establishing *in vitro* cellular models in order to study neurological or psychiatric diseases, as well as for the development of cell therapy-based strategies for certain neurological conditions.

The phenotypic transition from stem cells to neural precursors represents a limiting and crucial step of the neural, and later neuronal and glial, differentiation process. However, despite many research efforts, current neural induction protocols remain unsatisfactory because, due to limited efficacy of the selection step, the resulting cell population is often heterogeneous and/or poorly reproducible and/or slow to obtain. Moreover, most existing protocols are incompatible with the implementation of "GMP" (Good Manufacturing Practice) processes because they rely on products whose exact composition is poorly defined (such as serum, "serum replacement" products etc.) or because they require a co-culture step with feeder cells of animal origin. Furthermore, because of the heterogeneity of the resulting neural population, an initial sorting or selection step is necessary in order to select the cells of interest before amplification of neural precursors is possible. This step is at best time-consuming, and is often deleterious to the cells.

Therefore, there is still an existing need in the art for a method of obtaining a homogenous population of neural precursors from stem cells.

The inventors have developed a cell culture medium and a method for obtaining a homogenous population of neural precursors using said medium which does not require any step of culture in the presence of animal products, which is compatible with GMPs and which does not require any sorting or selection step.

### SUMMARY OF THE INVENTION

The invention relates to a culture medium comprising an inhibitor of the BMP signaling pathway; and an inhibitor of the TGF/activin/nodal signaling pathway, as defined in the claims.

The invention also relates to a method for producing a population of neural precursors wherein said method comprises the step of culturing pluripotent cells with the culture medium of the invention, as defined in the claims.

### DETAILED DESCRIPTION OF THE INVENTION

The present invention relates to a culture medium comprising an inhibitor of the BMP signaling pathway and an inhibitor of the TGF/activin/nodal signaling pathway, as defined in the claims.

The term "culture medium" as used herein refers to a liquid medium suitable for the in vitro culture of mammalian cells. Typically, the culture medium of the invention contains:
- a source of carbon as energy substrate, such as glucose, galactose or sodium pyruvate;
   - essential amino-acids;
   - vitamins, such as biotin, folic acid, B12...;
   - at least a purine and a pyrimidine as nucleic acid precursors;
   - inorganic salts;
   - a molecule known to limit natural ageing, such as selenium;
   - an antioxidant, such as glutathione reduced (GSH), Ascorbic acid, or enzymes involved in Reactive Oxygen Species detoxification, such as catalase or superoxyde dismutase;
   - a phospholipid precursor, such as choline, inositol or cholesterol derivative such as corticosterone;
   - an unique fatty acid, such as linoleic acid, linolenic acid and/or lipoic acids;
   - a carrier protein, such as Albumin and/or Heparin;
   - optionally, other proteins or peptides, such as insulin and/or transferrin and/or an agonist of the IGF-1 receptor.

The culture medium may also contain pH buffers in order to maintain the pH of the medium at a value suitable for cell growth.

The culture medium of the invention may be based on a commercially available medium such as DMEM/F12 from Invitrogen or a mixture of DMEM/F12 and Neurobasal in a 1:1 ratio (also from Invitrogen).

The culture medium of the invention may also comprise various supplements such as B-27 supplement (Invitrogen) and N2 supplement (also from Invitrogen).

The B27 supplement contains, amongst other constituents, SOD, catalase and other anti-oxidants (GSH), and unique fatty acids, such as linoleic acid, linolenic acid, lipoic acids.

The N2 supplement can be replaced with the following cocktail: transferrin (10g/L), insulin (500mg/L), progesterone (0.63mg/L), putrescine (1611 mg/L) and selenite (0.52mg/L).

The term "N2B27" refers to the medium described in Ying et al., 2003, in Lowell et al., 2006 and in Liu Y et al., 2006. N2B27 comprises DMEM/F12 and Neurobasal media in a 1/1 ratio, N2 supplement (1/100), B27 supplement (1/50) and beta-mercaptoethanol (1/1000). It is available, for example, under reference SCS-SF-NB-02 from Stem Cell Sciences UK Ltd.

In a preferred embodiment, the culture medium of the invention consists essentially of N2B27 medium, an inhibitor of the BMP signaling pathway and an inhibitor of the TGF/activin/nodal signaling pathway, as defined in the claims.

Typically, the culture medium of the invention is free of serum and free of serum extract.

In a preferred embodiment, the culture medium of the invention is free of animal-derived substances. In a preferred embodiment, the culture medium of the invention consists essentially of synthetic compounds, compounds of human origin and water. Advantageously, said culture medium can be used for culturing cells according to good manufacturing practices (under "GMP" conditions).

The term "'inhibitor of the BMP signaling pathway" as used herein refers to any compound, natural or synthetic, which results in a decreased activation of the BMP signaling pathway, which is the series of molecular signals generated as a consequence of any member of the BMP (bone morphogenetic protein) family binding to a cell surface receptor. Typically, an inhibitor of the BMP signaling pathway provokes a decrease in the levels of phosphorylation of the proteins Smad 1, 5 and 8 (Gazzero and Minetti, 2007).

The skilled person in the art knows how to assess whether a given compound is an inhibitor of the BMP signaling pathway. Typically, a compound is deemed to be an inhibitor of the BMP signaling pathway if, after culturing cells in the presence of said compound, the level of phosphorylated Smad 1, 5 or 8 is decreased compared to cells cultured in the absence of said compound. Levels of phosphorylated Smad proteins can be measured by Western blot using antibodies specific for the phosphorylated form of said Smad proteins.

The inhibitor of the BMP signaling pathway may be a BMP antagonist or a molecule which inhibits any downstream step of the BMP signaling pathway. The inhibitor of the BMP signaling may be a natural or a synthetic compound. When the inhibitor of the BMP signaling pathway is a protein, it may be a purified protein or a recombinant protein or a synthetic protein.

Many methods for producing recombinant proteins are known in the art. The skilled person can readily, from the knowledge of a given protein's sequence or of the nucleotide sequence encoding said protein, produce said protein using standard molecular biology and biochemistry techniques.

According to the present invention, the inhibitor of the BMP signaling pathway is selected from the group consisting of noggin, and fragments thereof which inhibit the BMP signaling pathway.

In a preferred embodiment, the inhibitor of the BMP signaling pathway is noggin. Noggin can be murine (mouse noggin exemplified by GenPept accession number NP_032737) or human noggin (human noggin exemplified by GenPept accession number EAW94528). It may be purified or recombinant. It may be in monomeric or dimeric form.

Recombinant noggin can be purchased from R&D Systems or Preprotech or can be produced using standard techniques as described above.

Typically, noggin is added to the culture medium of the invention in a concentration ranging from 100 to 600 ng/ml, preferably from 200 to 500 ng/ml, from 250 to 450 ng/ml, even more preferably at about 400 ng/ml.

In another embodiment, the inhibitor of the BMP signaling pathway is selected from the group of inhibitory Smad 6 (I-Smad 6) and inhibitory Smad 7 (I-Smad 7).

The term "'inhibitor of the TGF/activin/nodal signaling pathway" as used herein refers to any compound, natural or synthetic, which results in a decreased activation of the TGF/activin/nodal signaling pathway, which is the series of molecular signals generated as a consequence of any member of the TGF/activin/nodal family binding to a cell surface receptor. Typically, an inhibitor of the TGF/activin/nodal signaling pathway provokes a decrease in the levels of of phosphorylation of the protein Smad 2 (Shi and Massagué, 2003).

The skilled person in the art knows how to assess whether a given compound is an inhibitor of the TGF/activin/nodal signaling pathway. Typically, a compound is deemed to be an inhibitor of the TGF/activin/nodal signalling pathway if, after culturing cells in the presence of said compound, the level of phosphorylated Smad 2 is decreased compared to cells cultured in the absence of said compound. Levels of phosphorylated Smad proteins can be measured by Western blot using antibodies specific for the phosphorylated form of said Smad proteins.

The inhibitor of the TGF/activin/nodal signaling pathway may be a TGF/activin/nodal antagonist or a molecule which inhibits any downstream step of the TGF/activin/nodal signaling pathway. The inhibitor of the TGF/activin/nodal signaling may be a natural or a synthetic compound. When the inhibitor of the TGF/activin/nodal signaling pathway is a protein, it may be a purified protein or a recombinant protein or a synthetic protein.

Methods for producing recombinant proteins are known in the art. The skilled person can readily, from the knowledge of a given protein's sequence or of the nucleotide sequence encoding said protein, produce said protein using standard molecular biology and biochemistry techniques.

In one embodiment of the invention, the inhibitor of the TGF/activin/nodal signaling pathway is selected from the group consisting of SB431542, Lefty-A and Cerberus and fragments of Lefty-A and Cerberus which inhibit the TGF/activin/nodal signaling pathway.

In a preferred embodiment, the inhibitor of the TGF/activin/nodal signaling pathway is SB431542. SB431542, or 4-(5-Benzol[1,3]dioxol-5-yl-4-pyrlidn-2-yl-1 H-imidazol-2-yl)-benzamide hydrate, can be purchased from Tocris and Sigma. Typically, SB431542 is added to the culture medium of the invention in a concentration ranging from 5 to 25 µM, preferably ranging from 10 to 20 µM, even more preferably at about 20 µM.

The invention also relates to a kit for cell culture comprising an inhibitor of the BMP signaling pathway and an inhibitor of TGF/activin/nodal signaling pathway as described above.

The invention also relates to a kit for cell culture comprising a medium base (such as N2B27 medium defined above), an inhibitor of the BMP signaling pathway and an inhibitor of TGF/activin/nodal signaling pathway as described above.

In a particular embodiment of the invention, the inhibitor of the TGF/activin/nodal signaling pathway and the inhibitor of the BMP signaling pathway are different molecules.

### Cell culture methods of the invention

Another aspect of the invention relates to an *in vitro* method for producing a population of neural precursors wherein said method comprises the step of culturing pluripotent cells with the culture medium as described above.

The step of culturing pluripotent cells with the culture medium of the invention shall be carried out for the necessary time required for the production of neural precursors. Typically, the culture of pluripotent cells with the medium of the invention shall be carried out for at least 5 days, preferably at least 7 days, even more preferably at least 10 days.

If necessary, the culture medium of the invention can be renewed, partly or totally, at regular intervals. Typically, the culture medium of the invention can be replaced with fresh culture medium of the invention every other day, for 10 days.

The term "pluripotent cells" as used herein refers to undifferentiated cells which can give rise to a variety of different cell lineages. Typically, pluripotent cells may express the following markers oct4, SOX2, Nanog, SSEA 3 and 4, TRA 1/81, see International Stem Cell Initiative recommendations, 2007.

In one embodiment, the pluripotent cells are human pluripotent cells.

In another embodiment, the pluripotent cells are non-human mammalian pluripotent cells.

In one embodiment, the pluripotent cells are stem cells.

Typically, said stem cells are embryonic stem cells.

Illustrative examples of pluripotent cells are human embryonic stem cells (hES cells), such as the one described in the following table:

| line | karyotype | passage available | country of origin | origin |
|---|---|---|---|---|
| **SA01** | 46XY | 25 | Sweden | Cellartis AB |
| **VUB01** | 46XY | 73 | Belgium | AZ-VUB Bruxel |
| **HUES 24,** | 46XY | 26 | USA | Harvard |
| **H1** | 46XY, 20q11.21 | 26 | USA | Wicell research Institute |
| **H9** | 46XX | 27 | USA | Wicell research Institute |
| **WT3** | 46XY | 35 | UK | UKSCB |

In one embodiment, the pluripotent cells are non-human embryonic stem cells, such a mouse stem cells.

In one embodiment, the pluripotent cells are induced pluripotent stem cells (iPS). Induced pluripotent stem cells (iPS cells) are a type of pluripotent stem cells artificially derived from a non-pluripotent, typically an adult somatic cell, by inducing a "forced" expression of certain genes. iPS cells were first produced in 2006 from mouse cells (Takahashi et al Cell 2006 126 :663-76) and in 2007 from human cells (Takahashi et al. Cell 2007 131-861-72, Yu et al. Science 2007 318 :1917).

In one embodiment, the pluripotent cells contain a genetic mutation responsible for a neurodegenerative genetic disease. Advantageously, in this embodiment, the population of neural precursors obtained from said pluripotent cells also contains said mutation and can therefore provide a good cellular model of the disease.

Typically, cells lines baring triplet mutations causing the following neurodegenerative diseases can be employed:

| **Triplet mutation** | **Disease** | **Line** | **Reference** |
|---|---|---|---|
| Huntingtin-CAG | Huntington's disease | VUB05 | K.sermon, AZ-VUB Bruxel BELGIUM |
| Ataxin7-CAG | Spino-cerebellar ataxia | SCA7 | K.sermon, AZ-VUB Bruxel BELGIUM |
| DMPK-CTG | Steinert's disease (DM1) | VUB03 | K.sermon, AZ-VUB Bruxel BELGIUM |
| | | VUB19 | |
| | | VUB24 | |

The term "neural precursors or neural stem cells" as used herein refers to cells which are engaged in the neural lineage and which can give rise to any cell of the neural lineage including neurons and glial cells. Typically, neural precursors express the following markers: SOX1, SOX2, PAX6, Nestin, N-CAM (CD56), see Tabar et al., 2005; Sun et al., 2008.

In one embodiment, the invention relates to an *in vitro* method for obtaining neural precursors comprising the steps of:
- culturing pluripotent cells, preferably ES cells, in the presence of feeder cells;
- preparing clusters of said pluripotent cells, preferably ES cells, and preferably in the presence of a Rock inhibitor, preferably Y27632;
- culturing said clusters in low attachment dishes in the absence of feeder cells for several hours, preferably between 4 and 10 hours, more preferably between 5 and 8 hours, even more preferably for about 6 hours in order to starve said pluripotent cells, preferably ES cells , from the influence of the feeder cells;
- plating the suspension in dishes coated with poly-ornithin and laminin in the presence of a Rock inhibitor;
- replacing the medium every other day for with the medium of the invention.

Protocols for culturing pluripotent cells are known in the art. For example, culture of hES cells can be performed as described in (Amit et al., 2000)

The term "feeder cells" refers to the layer of cells, typically inactivated mouse fibroblasts, which are used for supporting the growth of ES cells. For example, the feeder cells can be the STO line available from ATCC.

The invention also discloses a population of neural precursors obtainable by a method as defined above.

Advantageously, the population of neural precursors is homogenous, i.e. it is not necessary to perform any sorting or selection to isolate the neural precursors from other contaminating cells. Typically, the population of neural stem cells has a purity of at least 95%, preferably 99%, even more preferably 100%.

The invention also describes a method for obtaining a population of neurons wherein said method comprises the steps of:
a. producing a population of neural precursors according to the method described above;
b. stabilizing an homogeneous population of neural stem cells
c. differentiating said population of neural stem cells into neurons.

The step consisting of differentiating neural stem cells into neurons can be carried according to techniques known to the skilled person (see for example Sun et al., 2008).
For example, neural precursors can be derived into neural stems cells by transfer into medium comprising Epithelial Growth Factor (EGF), Fibroblast Growth Factor 2 (FGF2) and Brain-Derived Neurotrophic Factor (BDNF), followed by plating onto plates coated with poly-ornithin/laminin and culture in the presence of BDNF.

The invention also describes a population of neurons obtainable by the method described above.

The term "neuron" as used herein refers to fully differentiated, post-mitotic cells of the neural lineage. Neurons express the following markers: beta-3 tubulin (TUJ1 antigen), Microtubule Associated Protein 2 (MAP2), HuC/D antigen.

Typically, the population of neurons has a purity of at least 40%, preferably 50%, even more preferably 60%.

The present invention also describes a pharmaceutical composition comprising the population of neural precursors or population of neurons as described above. The pharmaceutical composition may generally include one or more pharmaceutically acceptable and/or approved carriers, additives, antibiotics, preservatives, adjuvants, diluents and/or stabilizers. Such auxiliary substances can be water, saline, glycerol, ethanol, wetting or emulsifying agents, pH buffering substances, or the like. Suitable carriers are typically large, slowly metabolized molecules such as proteins, polysaccharides, polylactic acids, polyglycollic acids, polymeric amino acids, amino acid copolymers, lipid aggregates, or the like. This pharmaceutical composition can contain additional additives such as mannitol, dextran, sugar, glycine, lactose or polyvinylpyrrolidone or other additives such as antioxidants or inert gas, stabilizers or recombinant proteins (e. g. human serum albumin) suitable for in vivo administration.

As used herein, the term "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

Another aspect of the disclosure relates to a population of neural precursors of the invention or a population of neurons as described above, for use in treating a neurodegenerative disease or a brain injury.

The invention also describes a method for treating a neurodegenerative disease or brain injury comprising the step of administering a pharmaceutically effective amount of a population of neural precursors of the invention or a population of neurons as described above to a patient in need thereof.

In the context of the invention, the term "treating" or "treatment", as used herein, refers to a method that is aimed at delaying or preventing the onset of a pathology, at reversing, alleviating, inhibiting, slowing down or stopping the progression, aggravation or deterioration of the symptoms of the pathology, at bringing about ameliorations of the symptoms of the pathology, and/or at curing the pathology.

As used herein, the term "pharmaceutically effective amount" refers to any amount of neural precursors or neurons according to the invention (or a population thereof or a pharmaceutical composition thereof) that is sufficient to achieve the intended purpose.

Effective dosages and administration regimens can be readily determined by good medical practice based on the nature of the pathology of the subject, and will depend on a number of factors including, but not limited to, the extent of the symptoms of the pathology and extent of damage or degeneration of the tissue or organ of interest, and characteristics of the subject (e.g., age, body weight, gender, general health, and the like).

For therapy, neural precursors, neurons and pharmaceutical compositions may be administered through intracerebral route. The dose and the number of administrations can be optimized by those skilled in the art in a known manner.

In one aspect, the neurodegenerative disease or brain injury is selected from the group consisting of retinopathy, Huntington's disease, Spino-cerebellar ataxia, Steinert's disease, Parkinson's disease, Alzheimer's disease and cerebral ischemia, Multiple sclerosis, Amyotrophic lateral sclerosis, Traumatic Brain Injuries.

Yet another aspect of the invention discloses a method for screening compounds having a neuroprotective and/or neurotoxic effect wherein said method comprises the steps of:
a) culturing a population of neural precursors or a population of neurons as defined above in the presence of a test compound;
b) comparing the survival of the cells cultured in step a) to that of a population of neural precursors or a population of neurons as defined above cultured in the absence of said test compound.

The term "neurotoxic" refers to a compound which provokes a decrease in the survival of neural precursors or neurons. A compound is deemed to have a neurotoxic effect if the number of viable cells cultured in the presence of said compound is lower than the number of viable cells cultured in the absence of said compound.

The term "neuroprotective" refers to a compound which results in an increase survival of neural precursors or neurons. A compound is deemed to have a neuroprotective effect if the number of viable cells cultured in the presence of said compound is higher than the number of viable cells cultured in the absence of said compound. Typically, the neuroprotective effect can be assayed in the absence of neurotrophic factors. Alternatively, the neuroprotective effect can be assayed in the presence of a known neurotoxic drug. Known neurotoxic drugs include, but are not limited to,

The invention will be further illustrated through the following example and figures.,

### FIGURES

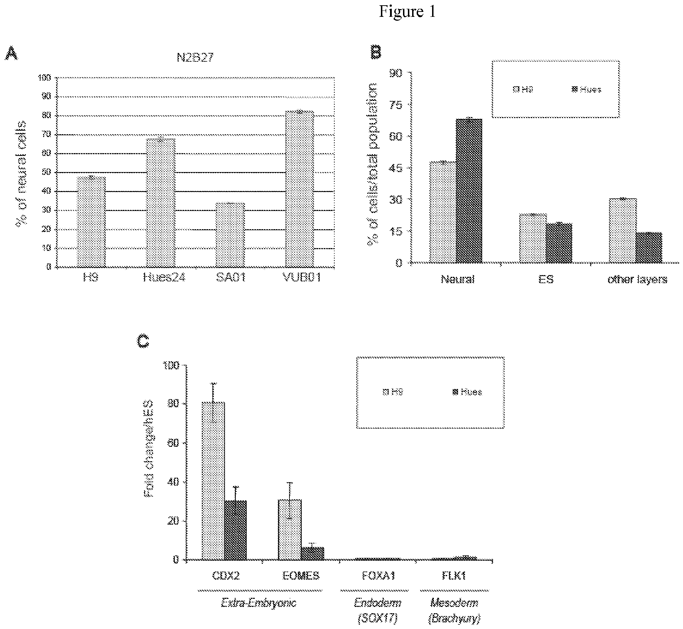

**FIGURE 1****: Neural differentiation in N2B27 "only"**
   **A-** FACS analyses of the efficiency of neural conversion obtained after 8 days in N2B27 for 4 representative hES cell lines.
   **B-** FACS analyses of the composition of the whole culture after 8 days of differentiation for two representative hES cell lines H9 and Hues 24.
   **C-** Characterization of the population committed to different fates by qPCR quantification of the levels of transcript of known markers of extra-embryonic tissues, mesoderm and endoderm primitive embryonic layers.

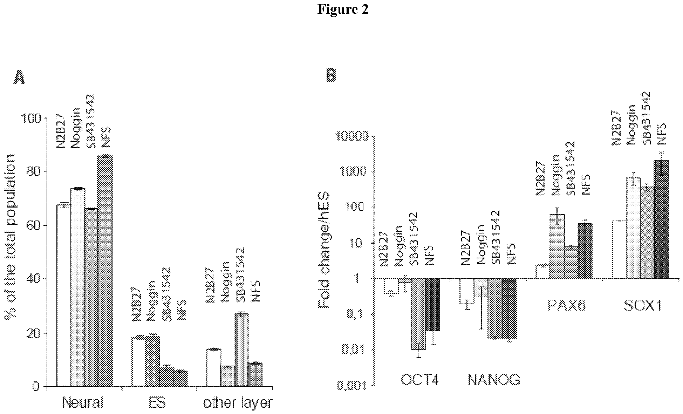

**FIGURE 2****: Effect of Noggin and SB431542 on neural differentiation of hES cells**
   **A-** FACS analyses of the composition of the whole culture after 8 days of differentiation in N2B27 alone or completed with Noggin, SB431542 or both (NFS).
   **B-** QPCR quantification of transcript specific of ES cells (Oct4 and Nanog) and early neural precursors (PAX6 and SOX1) for each condition of differentiation.

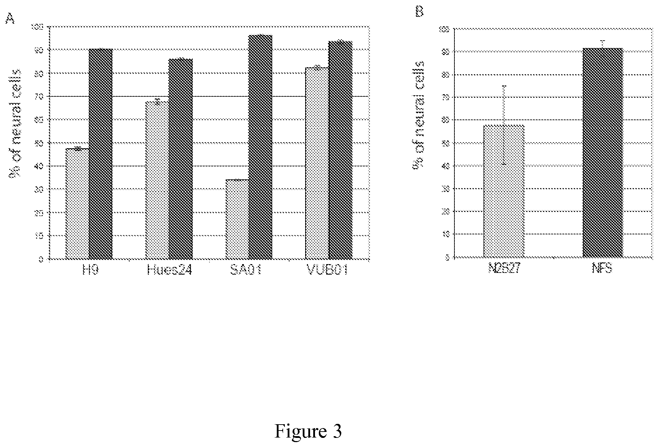

**FIGURE 3****: Robustness of the efficiency of the NFS medium on neural differentiation of hES cells**
   **A-** Comparison of the efficiency of N2B27 and NFS media on neural differentiation by FACS analyses (analyses line by line).
   **B-** Summary of the hES line on which NFS medium has been successfully used (average of the four lines).
**FIGURE 4****: Derivation, amplification and terminal differentiation of neural stem cells from neural tube-like structure obtained after differentiation of hES cells in NFS medium**
   **A-** Summary of the protocol of derivation of the stable and homogeneous population of Neural Stem Cells
   **B to D-** Typical morphologies that can be observed after the transfer of neural precursor cells (NEP cells) obtained by differentiation in NFS medium in a neural stem cells (NSC) amplification medium. **B-** After 2 days, NSC start to migrate from the neural tube-like structures (rosettes). **C-** homogeneous population of NSC obtained after a couple of passage. **D-** post mitotic neurons obtained after 2 weeks of starvation of NSC from EGF and FGF mitogenic activities.

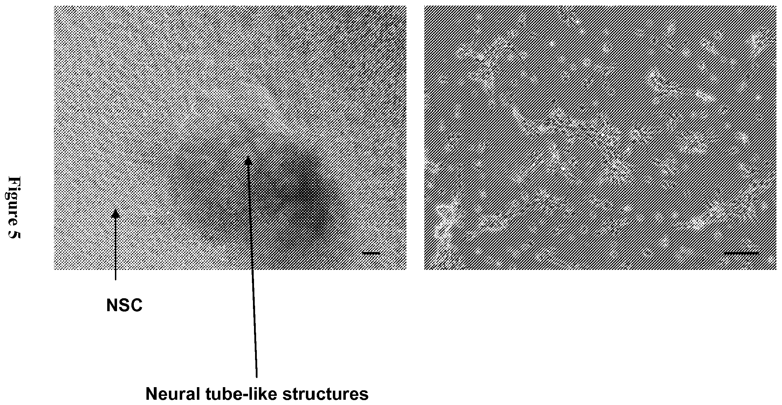

**FIGURE 5****: Differentiation of Induced Pluripotent Stem Cells (iPS) into neural stem cells (NCS) in NFS medium**
   Left panel: iPS treated for 10 days with NFS medium lead to neural-like structures and neural stem cells (NSC).
   Right panel: Morphology of iPS-derived NSC at passage 1.

### EXAMPLE

### MATERIAL AND METHODS

### Media and cytokines

N2B27 medium was described in Ying et al., 2003. N2B27 was a mixture of DMEM-F12/Neurobasal 1:1, N2 supplement (1:100°), B27 supplement (1:50°) both obtained from Invitrogen. NFS was composed of N2B27, Noggin (range of concentration between 200ng and 500ng/ml, from RD Systems or Preprotech.), SB431542 (between 10 and 20µM, from Tocris), 5ng/ml FGF2 (Preprotech.). Rock inhibitor Y27632 was from Calbiochem, EGF and BDNF were from RD systems.

### Human ES Cell Culture (illustrative example)

Human ES cells (Hues 24, XY, and H9, XX, WiCell Research Institute) were maintained on a layer of inactivated mouse fibroblasts (STO line from ATCC). The hES cells were cultured in DMEM/F12/Glutamax supplemented with 20% knockout serum replacement (KSR), 1 mM nonessential amino acids, 0.55 mM 2-mercaptoethanol, and 10 ng/ml recombinant human FGF2 (all from Invitrogen). Cultures were fed daily and manually passaged every 5-7 days. The cells were used between passages 40 and 60.

### iPS cell culture

Induced pluripotent stem cell line GMO3862 was obtained in the laboratory according to the reprogramming technique described in Takahashi et al. 2007. Briefly, fibroblasts were transduced with retroviral vectors expressing c-myc, Oct-4, Sox2 and Klf4.

### Neural induction using NFS Medium

Human ES cell cultures or iPS cultures reaching 70-80% confluence were used to perform neural induction using NFS medium. Colonies were cut in pieces and manually detached in N2B27 or NFS medium completed with the Rock-inhibitor Y27632 (10µM, Calbiochem). Clusters were transferred for 6h in a low attachment Petri dish in order to completely starve them from the influence of the feeders. Finally, the hES or iPS suspension was plated in the same medium at a 1:1 ratio in culture dishes pre-coated with poly-ornithin and laminin (2µg/ml, Sigma). The day after and then every other day, the medium was changed for NFS medium without Y27632.

### Neural stem cells derivation and terminal differentiation into neurons

Ten days after the induction in NFS medium, neural precursors were transferred in an amplification medium composed of N2B27, EGF/FGF2 (10ng/ml) and BDNF (Brain- derived Neurotrophic Factor, 20ng/ml) without passaging, in order to allow the neural stem cells (NSC) to migrate outside the neuro-epithelial structures. When the NSC culture was at full confluence, the cells were passaged using trypsin and replated on poly-ornithin/laminin at a ratio of 1:2 in EGF/FGF2/BDNF medium. Terminal differentiation into neurons was induced by platting the NSC on poly-ornithin/laminin at a density of 50,000 cells /cm² in N2B27 + BDNF (without EGF and FGF2). Analyses were performed 2 weeks after the terminal differentiation.

### FACS analysis

Cells were collected using trypsin and fixed with 2% PFA for 15 min at 4°C. Permeabilization was performed using a PBS/0.1% saponin solution 10 min at RT. The same solution was then used to dilute primary antibodies as followed: mouse monoclonal OCT4 antibody linked to phycoerythrin (oct4-PE, 1:10°, BD Biosciences) and polyclonal rabbit anti-SOX2 antibody (1:500°, Chemicon). Cells were exposed to the mixture of primary antibodies 45 min at RT. An additional incubation with anti-rabbit secondary antibodies linked to AlexaFluo 488 was performed during 30 min at RT in order to detect SOX2 unconjugated antibodies. FACS analysis was performed using a Becton Dickinson FACScalibur™ flow cytometer.

### Immuno-cytochemistry (ICC)

Cells were fixed with 4% PFA for 15 min at 4°C then permeabilized using a PBS/0.3% Triton X100 solution, 10 min at RT. Incubation with primary antibodies was performed as followed, overnight at 4°C in PBS/TX100: rabbit polyclonal anti-PAX6 (1:800°, Covance), rabbit polyclonal anti-SOX2 (1:1000°), mouse monoclonal anti-OCT4 (1:200°, Santa-cruz biotech.), mouse monoclonal anti-CytoKeratin 18 (1:50°, Abcam), mouse monoclonal anti-PAX3 (1:50°, RD Systems), mouse monoclonal anti-TUJ1 and mouse monoclonal anti-MAP2 (1:1000°, both from Covance). AlexaFluor secondary antibodies and DAPI counterstaining were applied for 1 h at room temperature. Detection was performed using a Zeiss Inverted microscope.

### Real-Time qPCR.

Total RNA was isolated using the RNeasy Mini Kit (Qiagen) according to the manufacturer's instructions. A total of 500 ng of RNA were reverse transcribed into cDNA with SuperScript III (Invitrogen) using random primers. Real-time Q-PCR was performed with SYBR Green as a probe on a LC480 Real-Time system (Roche). Quantification was performed at a threshold detection line (Ct value). The Ct of each target gene was normalized against that of the cyclophilin as a housekeeping gene. The 2-ΔΔCt method was used to determine the relative level of expression of each gene. Data were expressed as mean ± SEM. Primer sequences are listed in follow.

| | |
|---|---|
| NA NOG F | ctccatgaacatgcaacctg (SEQ ID No: 1) |
| NA | ctcgctgattaggctccaac (SEQ ID No: 2) |
| NOG R | |
| oct 4F | cttgctgcagaagtgggtggaggaa (SEQ ID No: 3) |
| oct 4R | ctgcagtgtgggtttcgggca (SEQ ID No: 4) |
| So x1 F | gatgcacaactcggagatca (SEQ ID No: 5) |
| So x1 R | gtccttcttgagcagcgtct (SEQ ID No: 6) |
| Pa x6 F | gccagcaacacacctagtca (SEQ ID No: 7) |
| Pa x6 R | tgtgagggctgtgtctgttc (SEQ ID No: 8) |

### RESULTS

### Efficiency of neural differentiation in N2B27 is highly variable depending of the hES cells line used. (illustrative example)

We first monitored the efficiency of neural differentiation obtained only by removing from the culture medium any known instructive signals for alternative cell fates. We used N2B27 medium, a defined medium previously developed to induce neural differentiation of both mouse and human embryonic stem cells in an adherent monolayer culture system (Ying et al., 2003; Lowell et al., 2006). In order to analyzed consistently differentiation of several naïve hES cell lines, we have develop a protocol of systematic counting where cells were stained using antibodies against OCT4 and SOX2 transcription factors and then, analysed using FACS technology. Cells expressing both OCT4 and SOX2 were considered as embryonic stem cells whereas cells expressing only SOX2 were counted as neural cells. Cells totally negative or expressing only OCT4 were designated as differentiated into other type of tissues or layers. FACS analysis was performed on 4 hES cell lines (Hues 24, H9, VUB01, SA01), 8 days after platting on laminin in N2B27. Neural differentiation was obtained with all cell lines, but the efficiency of differentiation appeared highly variable depending of the cell line used, ranging from 33.84% for SA01 line to 82.33% for VUB01 line (Figure 1-A). This indicated that the simple removal of instructive factors from the culture medium of monolayer cultures of ES cells is not sufficiency to optimally induce commitment to the neural lineage.

We then decided to further characterize the culture obtained after 8 days of differentiation in N2B27 medium. FACS quantification and immuno-cytochemistry analyses were performed on two representative cell lines H9 and Hues 24 (Figure 1 B). These lines were chosen because their potential of differentiation was in the middle range and because they were representative of each sex, H9 being a female line and Hues 24 being a male line. For each cell line, next to neural cells (Oct4-/SOX2+/PAX6+), ES cells resistant to differentiation (OCT4+/SOX2+/PAX6-) were detected (22.56% for H9 line and 18.43% for Hues24 line). In addition, significant portion of the culture consist of cells committed to alternative fates (30.05% for H9 line, 13.95% for Hues24 line). In order to characterize these alternative types of differentiation, expression of markers of the three primitive germ layers and of extra-embryonic tissue were quantified by qPCR (Figure 1 C). Markers of primitive endoderm (SOX17 and FOXA1) or primitive mesoderm (Brachyury and FLK1) were not detected, indicating that cells were not committed toward other primitive embryonic layers. In contrast, strong levels of transcripts of 2 markers of extra-embryonic tissues, CDX2 and Eomes, were present. Taken together, qualitative and quantitative analyses shown that the final population of cells obtained after differentiation of hES as an adherent monolayer in N2B27 is composed of a mixture of neural precursors, undifferentiated ES cells and cells of extra-embryonic origin.

### BMP and TGFbeta inhibitors (Noggin and SB431542) have non redundant, complementary effects on the induction of neural differentiation from hES cells. (illustrative example)

In order to explain this heterogeneity, we hypothesised that, despite the absence of instructive signals coming from the medium, autocrin or paracrin stimulations of intra-cellular pathways may exist in the small clumps/colonies of ES cells that are sufficient to maintain self renewing or induce extra-embryonic differentiation. Because the concomitant presence of undifferentiated ES cells and extra-embryonic tissue can be a typical signature of persistent SMAD activation, we focused on the two pathways known to induce such activations: the TGF beta pathway, also known as Activin/Nodal pathway, and the BMP pathway.

We first investigated the consequence of a pharmacological inhibition of the TGF beta pathway by SB 431542, a chemical known to specifically block the Activin/Nodal receptor-dependant SMAD activation. This molecule has been shown to increase the expression of neurectoderm makers like Nestin and NCAM in a system based on the formation of embryonic bodies. A eight days treatment of the adherent monolayer of cells with SB431542 in N2B27 didn't increase robustly the expression of early neural markers like PAX6 and SOX1 when mesured by qPCR or the proportion of neural cells (Oct4-/PAX6+) when quantified by FACS (figure 2). However this treatment induced a drastic differentiation of ES into an alternative fate which was not due to an increased induction of extra-embryonic tissues, as shown by qPCR analyses of CDX2 and Eomes transcripts, or a *de novo* commitment into one of the other primitive embryonic layers since expression of the 2 mesoderm markers FLK1 and Brachyury and of 2 endoderm markers SOX17 and FOXA1 were not detected. In contrast, cells commited to the two other kind of progeny deriving from the primitive ectoderm, namely neural crest cells (PAX3+/SOX10+) and keratinocytes (expressing Cytokeratins 8 and 18), were detected both by analysing the levels of each transcript by qPCR or checking the expression of PAX3 and CK18 by ICC. This indicated that SB431542, and more generally inhibition of TGF beta pathway, didn't promote specifically neural differentiation but is necessary to induce the differentiation of ES cells and to promote their entry into primitive ectoderm lineage.

In parallel, we investigate the impact of the inhibition of the BMP pathway by its natural inhibitor Noggin (figure 2). If an 8 days treatment of the adherent monolayer with Noggin induced a slight increased of the expression of neural markers (qPCR) and of the percentage of neural cells (FACS analyses), the main effect of this cytokine was a complete blockage of the differentiation of hES cells into an alternative fate that the neural lineage. As a result, next to the neural cells, a large number of ES cells expressing Oct4 and NANOG transcription factors remained in the culture after 8 days of treatment. Cells differentiated into extra-embryonic tissues, mesoderm, endoderm, keratinocytes or neural crest cells were not detected nor by qPCR analyses or ICC. Since the single blockage of BMP pathway appeared to act mainly as a blocker of non-neural fate but it's not sufficient by itself to trigger hES differentiation, we decided to combine this "restrictive" effect with the "differentiated" effect of SB431542

When the two inhibitors were combined (figure 2), we observed a robust and synchronized neural differentiation of hES cells since over 90% of cells committed to neural lineage were detected by FACS (Oct4-/SOX2+). Qualitative analyses by ICC have shown that the culture was homogeneously organized into neural tube-like structures or "rosettes" composed of cells expressing the neural plate marker PAX6. In agreement with these qualitative observations, the combined treatment induced the highest expression of transcripts the two neural commitment markers PAX6 and SOX1 as measured by qPCR. Minimal levels of commitment markers for alternative fates deriving from the primitive ectoderm were detected. Taken together, these results indicated that the combination of the two inhibitors (NFS medium) was necessary to induce a synchronized and efficient differentiation of ES cells into neural precursors. To date, NFS medium was tested on a total of 10 hES lines, including lines naturally bearing monogenic diseases causal mutations with the same efficiency i.e. over 90% of neural cells after 8-10 days of differentiation.

### This synchronization allows the direct derivation of neural stem cells without the need of sorting or selection

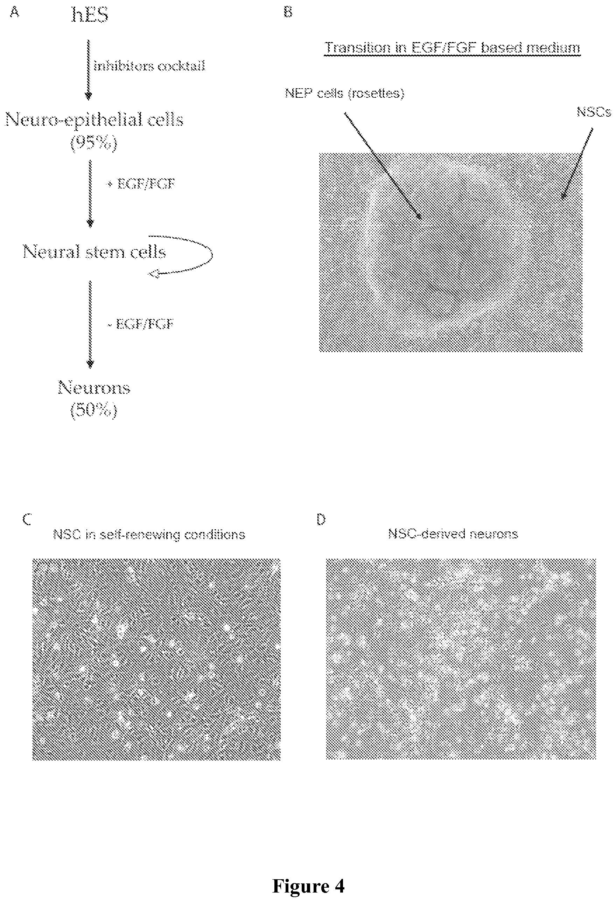

Once the neural precursor cells (PAX6+/SOX1+/SOX2+) were obtained (after 8-10 days of differentiation), the neural tube-like structures were transferred into another defined medium more appropriated to start their amplification and further stabilization as neural "stem cells" (NSC, Figure 4A). This medium was described previously (Conti et al., 2005) and its amplification properties were based on the used of a combination of two mitogens namely EGF (*Epidermal Growth factor)* and FGF2 *(Fibroblast Growth factor 2)* and the optional addition of the survival factor BDNF (*Brain-derived Growth Factor).* After a couple of day, neural stem cells started to migrate from the rosette structures and started to be morphologically identifiable (Figure 1 B). When reaching confluence, the culture was passed using trypsin to detach the cells and re-seed on poly-ornithin/laminin substrates at a ratio of 1 in 2. After 2 or 3 passages, a stable and homogeneous population of neural stem cells was obtained. These cells expressed the same markers than the ones described in the literature for hES cells-derived neural stem cells obtained using alternative methods or radial glia-like cells derived from foetuses, like SOX2, Nestin and Blbp. In order to test their potential to give rise to neurons, NSC were platted at low density (usually between 50,000 and 100,000 cells/cm²) on poly-ornithin/laminin substrates in N2B27 medium + 20 ng/ml of BDNF. Neurites outgrowth starts within the first 24 hours following proliferation factors withdrawal, and neurons are clearly identifiable after 4 days. The highest rate of differentiation is obtained after about 10 days (over 60% of the entire population). The resulting culture is composed of a mixture of inhibitory GABAergic and excitatory glutamatergic neurons without defined regional identity. We have also observed the spontaneous differentiation of NSC into GFAP positive astrocytes, demonstrating that neural stem cells were multipotent.

As shown in Figure 5, NSC were also obtained after differentiation of iPS cells in NFS medium.

### REFERENCES

Throughout this application, various references describe the state of the art to which this invention pertains.
Amit M, Carpenter MK, Inokuma MS, Chiu CP, Harris CP, Waknitz MA, Itskovitz-Eldor J, Thomson JA. Clonally derived human embryonic stem cell lines maintain pluripotency and proliferative potential for prolonged periods of culture. Dev Biol. 2000 Nov 15;227(2):271-8.
Conti L, Pollard SM, Gorba T, Reitano E, Toselli M, Biella G, Sun Y, Sanzone S, Ying QL, Cattaneo E, Smith A. Niche-independent symmetrical self-renewal of a mammalian tissue stem cell. PLoS Biol. 2005 Sep;3(9):e283.
Gazzerro E, Minetti C.Potential drug targets within bone morphogenetic protein signaling pathways. Curr Opin Pharmacol. 2007 Jun;7(3):325-33.
International Stem Cell Initiative. Characterization of human embryonic stem cell lines by the International Stem Cell Initiative. Nat Biotechnol. 2007 Jul;25(7):803-16.
Liu Y, Song Z, Zhao Y, Qin H, Cai J, Zhang H, Yu T, Jiang S, Wang G, Ding M, Deng H. A novel chemical-defined medium with bFGF and N2B27 supplements supports undifferentiated growth in human embryonic stem cells. Biochem Biophys Res Commun. 2006 Jul 21;346(1):131-9.
Lowell S, Benchoua A, Heavey B, Smith AG. Notch promotes neural lineage entry by pluripotent embryonic stem cells. PLoS Biol. 2006 May;4(5)
Shi Y, Massagué J. Mechanisms of TGF-beta signaling from cell membrane to the nucleus. Cell. 2003 Jun 13;113(6):685-700.
Sun Y, Pollard S, Conti L, Toselli M, Biella G, Parkin G, Willatt L, Falk A, Cattaneo E, Smith A. Long-term tripotent differentiation capacity of human neural stem (NS) cells in adherent culture. Mol Cell Neurosci. 2008 Jun;38(2):245-58
Tabar V, Panagiotakos G, Greenberg ED, Chan BK, Sadelain M, Gutin PH, Studer L. Migration and differentiation of neural precursors derived from human embryonic stem cells in the rat brain. Nat Biotechnol. 2005 May;23(5):601-6.
Takahashi K. and Yamanaka S. Induction of pluripotent stem cells from mouse embryonic and adult fibroblast cultures by defined factors. Cell. 2006 Aug 25;126(4):663-76
Takahashi K, Tanabe K, Ohnuki M, Narita M, Ichisaka T, Tomoda K, Yamanaka S. Cell. 2007 Nov 30;131 (5):861-72.
Ying QL, Stavridis M, Griffiths D, Li M, Smith A. Conversion of embryonic stem cells into neuroectodermal precursors in adherent monoculture. Nat Biotechnol. 2003 Feb;21(2):183-6.
Yu J, Vodyanik MA, Smuga-Otto K, Antosiewicz-Bourget J, Frane JL, Tian S, Nie J, Jonsdottir GA, Ruotti V, Stewart R, Slukvin II, Thomson JA. Induced pluripotent stem cell lines derived from human somatic cells. Science. 2007 Dec 21;318(5858):1917-20.

## Claims

1. A culture medium comprising :
a) noggin or fragments thereof which inhibit the BMP signalling pathway; and
b) an inhibitor of the Transforming Growth Factor (TGF)/activin/nodal signaling pathway selected from the group consisting of SB431542, Lefty-A and Cerberus and fragments of Lefty-A and Cerberus which inhibit the TGF/activin/nodal signaling pathway.

2. A culture medium according to claim 1, wherein said culture medium further comprises :
a) a source of carbon;
b) essential amino-acids;
c) vitamins:
d) a purine and a pyrimidine;
e) inorganic salts;
f) selenium;
g) an antioxidant;
h) a phospholipid precursor;
i) an unique fatty acid; and
j) a carrier protein.

3. An *in vitro* method for producing a population of neural precursors wherein said method comprises the step of culturing pluripotent cells with the culture medium as defined in any one of claims 1 to 2.

4. An *in vitro* method according to claim 3 wherein said pluripotent cells are human pluripotent cells.

5. An *in vitro* method according to claim 3 or 4 wherein said pluripotent cells are stem cells.

6. An *in vitro* method according to claim 5 wherein said stem cells are embryonic stem cells.

7. An *in vitro* method according to claim 3 or 4 wherein said pluripotent cells are induced pluripotent cells (IPS).

8. A kit for cell culture comprising:
a) noggin or fragments thereof which inhibit the BMP signalling pathway and
b) an inhibitor of the Transforming Growth Factor (TGF)/activin/nodal signaling pathway selected from the group consisting of SB431542, Lefty-A and Cerberus and fragments of Lefty-A and Cerberus which inhibit the TGF/activin/nodal signaling pathway.

9. A kit for cell culture according to claim 8, further comprising a medium base.

10. An *in vitro* method according to any of claims 3-7, wherein said pluripotent cells are cultured with the medium as defined in claim 1 or 2 for at least 5 days.

11. An *in vitro* method according to any of claims 3-7, wherein said pluripotent cells are cultured with the medium as defined in claim 1 or 2 for at least 7 days.

12. An *in vitro* method according to any of claims 3-7, wherein said pluripotent cells are cultured with the medium as defined in claim 1 or 2 for at least 10 days.

## Patentansprüche

1. Ein Kulturmedium enthaltend:
a) Noggin oder Fragmente davon, welche den BMP-Signaltransduktionsweg inhibieren; und
b) einen Inhibitor des Transformierenden Wachstumsfaktor (TGF)/Activin/Nodal-Signal-Transduktionweges, ausgewählt aus der Gruppe, bestehend aus SB431542, Lefty-A und Cerberus und Fragmenten von Lefty-A und Cerberus, welche den TGF/Activin/Nodal-Signaltransduktionweg inhibieren.

2. Ein Kulturmedium nach Anspruch 1, wobei das Kulturmedium weiterhin umfasst:
a) eine Kohlenstoffquelle;
b) essentielle Aminosäuren;
c) Vitamine;
d) ein Purin und ein Pyrimidin;
e) anorganische Salze;
f) Selen;
g) ein Antioxidans;
h) einen Phospholipid-Präkursor;
i) eine "einzigartige" Fettsäure; und
j) ein Trägerprotein.

3. Ein "in vitro "-Verfahren zur Herstellung einer Population von neuralen Präkursoren, wobei dieses Verfahren den Schritt der Kultivierung pluripotenter Zellen mit dem Kulturmedium, wie in einem der Ansprüche 1 bis 2 definiert, umfasst.

4. Ein "in vitro"-Verfahren nach Anspruch 3, wobei diese pluripotenten Zellen humane pluripotente Zellen sind.

5. Ein "in vitro"-Verfahren nach Anspruch 3 oder 4, wobei diese pluripotenten Zellen Stammzellen sind.

6. Ein "in vitro"-Verfahren nach Anspruch 5, wobei diese Stammzellen embryonale Stammzellen sind.

7. Ein "in vitro"-Verfahren nach Anspruch 3 oder 4, wobei diese pluripotenten Zellen induzierte pluripotente Zellen (IPS) sind.

8. Ein Kit für die Zellkultur umfassend:
a) Noggin oder Fragmente davon, welche den BMP-Signaltransduktionsweg inhibieren, und
b) einen Inhibitor des Transformierenden Wachstumsfaktor (TGF)/Activin/Nodal-Signaltransduktionsweges, ausgewählt aus der Gruppe, bestehend aus SB431542, Lefty-A und Cerberus und Fragmenten von Lefty-A und Cerberus, welche den TGF/Activin/Nodal-Signaltransduktionsweg inhibieren.

9. Ein Kit für die Zellkultur nach Anspruch 8, weiterhin umfassend eine Mediumbase.

10. Ein "in vitro"-Verfahren nach einem der Ansprüche 3 bis 7, wobei diese pluripotenten Zellen mit dem Medium, wie in Anspruch 1 oder 2 definiert, für mindestens 5 Tage kultiviert werden.

11. Ein "in vitro"-Verfahren nach einem der Ansprüche 3 bis 7, wobei diese pluripotenten Zellen mit dem Medium, wie in Anspruch 1 oder 2 definiert, für mindestens 7 Tage kultiviert werden.

12. Ein "in vitro"-Verfahren nach einem der Ansprüche 3 bis 7, wobei diese pluripotenten Zellen mit dem Medium, wie in Anspruch 1 oder 2 definiert, für mindestens 10 Tage kultiviert werden.

## Revendications

1. Milieu de culture comprenant :
a) une noggine ou des fragments de celle-ci qui inhibent la voie de signalisation de la BMP ; et
b) un inhibiteur de la voie de signalisation du facteur de croissance transformant (TGF)/activine/nodal choisi dans le groupe constitué de SB431542, de Lefty-A et de Cerberus et des fragments de Lefty-A et de Cerberus qui inhibent la voie de signalisation du TGF/activine/nodal.

2. Milieu de culture selon la revendication 1, dans lequel ledit milieu de culture comprend en outre :
a) une source de carbone ;
b) des acides aminés essentiels ;
c) des vitamines ;
d) une purine et une pyrimidine ;
e) des sels inorganiques ;
f) du sélénium ;
g) un antioxydant ;
h) un précurseur de phospholipide ;
i) un acide gras unique ; et
j) une protéine de support.

3. Procédé *in vitro* de production d'une population de précurseurs neuronaux dans lequel ledit procédé comprend l'étape de culture de cellules pluripotentes avec le milieu de culture tel que défini dans l'une quelconque des revendications 1 à 2.

4. Procédé *in vitro* selon la revendication 3 dans lequel lesdites cellules pluripotentes sont des cellules pluripotentes humaines.

5. Procédé *in vitro* selon la revendication 3 ou 4 dans lequel lesdites cellules pluripotentes sont des cellules souches.

6. Procédé *in vitro* selon la revendication 5 dans lequel lesdites cellules souches sont des cellules souches embryonnaires.

7. Procédé *in vitro* selon la revendication 3 ou 4 dans lequel lesdites cellules pluripotentes sont des cellules pluripotentes induites (IPS).

8. Kit pour culture de cellules comprenant :
a) une noggine ou des fragments de celle-ci qui inhibent la voie de signalisation de la BMP ; et
b) un inhibiteur de la voie de signalisation du facteur de croissance transformant (TGF)/activine/nodal choisi dans le groupe constitué de SB431542, de Lefty-A et de Cerberus et des fragments de Lefty-A et de Cerberus qui inhibent la voie de signalisation du TGF/activine/nodal.

9. Kit pour culture de cellules selon la revendication 8, comprenant en outre une base de milieu.

10. Procédé *in vitro* selon l'une quelconque des revendications 3 à 7, dans lequel lesdites cellules pluripotentes sont cultivées avec le milieu tel que défini dans la revendication 1 ou 2 pendant au moins 5 jours.

11. Procédé *in vitro* selon l'une quelconque des revendications 3 à 7, dans lequel lesdites cellules pluripotentes sont cultivées avec le milieu tel que défini dans la revendication 1 ou 2 pendant au moins 7 jours.

12. Procédé *in vitro* selon l'une quelconque des revendications 3 à 7, dans lequel lesdites cellules pluripotentes sont cultivées avec le milieu tel que défini dans la revendication 1 ou 2 pendant au moins 10 jours.
